# EUROPEAN PATENT APPLICATION

(11) **EP 4 397 332 A2**
(43) Date of publication of application: **10.07.2024**
(21) Application number: 24156085.3
(22) Date of filing: 08.03.2023
(51) Int. Cl.: A61L 31/14

(54) **BIODEGRADABLE, NON-POROUS MEDICAL DEVICE AND METHODS FOR MAKING AND USING THE SAME**

(30) Priority: 09.03.2022 EP 22161125
(62) Divisional of application: 23710700.8
(71) Applicant: Octapharma AG, 8853 Lachen (CH)
(72) Inventor: Tschetschkowitsch, Klaus, 1100 Wien (AT); Covini, Caroline, 1100 Wien (AT)
(74) Representative: Ullrich & Naumann PartG mbB

(57) **Abstract**

The present invention relates to biodegradable, non-porous medical devices and particular to such devices comprising a sheet-like structural layer and a multilayered water-activatable adhesive component. The invention further relates to methods of making and using the biodegradable, non-porous medical devices. Embodiments of the invention have been particularly developed for making flexible yet burst-resistant biodegradable, non-porous medical devices, where the devices' adhesive component renders them particularly useful as a hemostat in the treatment and/or prevention of mild to severe as well as arterial bleedings. Embodiments of the invention will be described hereinafter with reference to these applications. However, it will be appreciated that the invention is not limited to this particular field of use.

## Description

### FIELD OF THE INVENTION

The present invention relates to biodegradable, non-porous medical devices and particular to such devices comprising a sheet-like structural layer and a multi-layered water-activatable adhesive component. The invention further relates to methods of making and using the biodegradable, non-porous medical devices.

Embodiments of the invention have been particularly developed for making flexible yet burst-resistant biodegradable, non-porous medical devices, where the devices' adhesive component renders them particularly useful as a hemostat in the treatment and/or prevention of mild to severe as well as arterial bleedings. Embodiments of the invention will be described hereinafter with reference to these applications. However, it will be appreciated that the invention is not limited to this particular field of use.

### BACKGROUND OF THE INVENTION

Any discussion of the background art throughout the specification should in no way be considered as an admission that such art is widely known or forms part of common general knowledge in the field.

Hemostats are known in the art as adjunctive measures to improve hemostasis during surgical procedures. In particular, hemostats are used for hemorrhage control when residual bleeding persists. Topical hemostats encompass a wide variety of products, typically configured for application in challenging surgical settings or for patients are affected by congenital or acquired coagulation disorders.

For example, *mechanical hemostats* comprising porcine gelatine, oxidized cellulose, bovine collagen and/or plant-derived polysaccharide spheres are known. Such hemostats act by initiating platelet activation and aggregation as well as by forming a matrix at the site of bleeding through which clotting is mediated.

Mechanical hemostats are regularly configured to absorb relatively large volumes of liquid, i.e. volumes several times the hemostat's own weight and are available in sponge, powder and granular forms. Necessarily, absorption of relatively large volumes of liquid leads to swelling of the hemostat, which, once applied, may lead to complications from compression effects exerted by the hemostat onto adjacent tissues. Further, mechanical hemostats are not biologically active and, therefore, are not suitable for use in patients with a coagulation disorder as they rely on the patient's own ability to form a fibrin clot to stem a bleed. However, whether the induction of clot formation alone - when mediated by mechanical hemostats - is sufficient to prevent leakages from injured tissues such as biliary leakage during liver surgery or pancreatic leakage during pancreas surgery and whether mechanical hemostats are appropriate sealants in this context remains a topic of discussion.

In contrast, hemostatic sealants, which as the nomenclature indicates are typically used as hemostats and as sealants are also known. Such sealants are usually low viscosity liquids that only polymerize at the site of injury, trauma or surgery, i.e. *in situ,* such as to form a solid film and to a-traumatically connect themselves and adjacent tissues through covalent polymerisation. In this group of hemostats, synthetic sealants comprising cyanoacrylates and/or polyethylene glycol-PEG and semisynthetic sealants comprising glutaraldehyde albumin-derived sealants are usually distinguished. The polymerisation reaction is a non-enzymatic, chemical reaction and is therefore independent of the blood coagulation cascade. Synthetic and semi-synthetic sealants are generally not biodegradable as such and most of the polymeric components ultimately need to be cleared from the patient's body. In this regard, it is noteworthy that safety considerations with PEG-based sealants include the risk of impaired renal function, due to the renal clearance of the polyethylene glycol.

PEG-based sealants are manufactured both as pads of different sizes and as flowing products. Given PEG's propensity to swell, caution must be taken when applying such sealants near compression-sensitive tissues such as nerves. While allergic reactions to PEG are still rare, such excipient allergies are becoming more prevalent and can be severe. Similarly, allergic reactions to albumin-containing devices are to be considered in addition to keeping the potential risk of transmission of communicable diseases or viruses from the human serum albumin component in mind.

While cyanoacrylate-based sealants are known to polymerise very quickly, this polymerisation reaction is an exothermic reaction, which can damage adjacent tissues through the heat it generates.

In addition to the above, adhesive hemostatic devices are known, which comprise fibrinogen and thrombin such as to actively participate at the end of the coagulation cascade to form a hemostatic fibrin clot. Such devices are available, for example, in liquid flowable form (fibrin glues) or in association with stiff collagen fleece (patch) and are particularly suitable for application in patients affected by congenital or acquired coagulation disorders. One aspect is the speed of hemostasis, the characteristics of fibrin clot and the agent's safety profile. In particular, a higher fibrinogen concentration tends to produce a stronger, but slow forming clot, whereas higher thrombin concentrations result in rapid clot formation, but the clot formed is not as strong.

Liquid fibrin adhesives are typically applied by means of a single or double syringe system such as to allow clot formation *in situ.* Safety concerns include the fact that when using syringe systems, particular care must be taken not to inject any of the liquid adhesive containing thrombin intravascularly because this can readily lead to thrombosis, hypotension and potentially even death. In addition, liquid fibrin adhesives are not well-suited to stem severe or even arterial bleeding due to the streaming effect of the flowing blood, i.e. they tend to be simply washed away before exerting their function.

In direct comparison, solid fibrin adhesives, which comprise a mechanical support such as that offered by a collagen or an oxidized cellulose matrix, do not suffer from the streaming effect and are therefore also used in the context of stronger bleeds. However, the mechanical support provided by the collagen or oxidized cellulose matrices of such patches is typically limited again, as these materials are regularly stiff and/or brittle and only have little flexibility or resistance to burst pressure.

Therefore, in order to be sufficiently stable to be handled by a surgeon as well as to withstand the pressures exerted by a severe bleed, these patches are often clearly three-dimensional structures such as sponges or relatively thick wads, i.e. regularly having a thickness in the range of one to several millimeters. Further, fibrin patches are often porous, which renders them less suitable as sealants in circumstances where leakages must be stopped quickly. In this regard, we note that the effectiveness of fibrin patches in the prevention of air leaks after thoracic surgery procedures and of pancreatic or bile leaks after resective interventions remains a matter of discussion in the field.

Accordingly, a need for improved biodegradable medical devices, which have a high degree of flexibility and high mechanical strength and which can easily be affixed to a site of injury or surgically-induced trauma such as to act in hemorrhage control stemming even severe bleeds are needed.

### SUMMARY OF THE INVENTION

It is an object of the present invention to overcome or ameliorate at least one of the disadvantages of the prior art, or to provide a useful alternative. In particular, it is an object of the present invention to provide improved biodegradable medical devices and to provide improved methods for making and using such devices, for example in the treatment of medical indications.

The present invention is *inter alia* based on the surprising finding that the advantageous flexibility and/or elasticity and/or mechanical strength of known plasma-based or cryoprecipitate-based films, when expressed as a function of the films' resistance to burst pressure, is not negatively impacted when such a films are used as the structural layer in a medical device suitable for use as a hemostat and comprising a particularly advantageous multi-layered adhesive component to affix the device to the site of injury or surgery induced trauma.

Accordingly, in a first aspect, the present invention relates to a biodegradable, non-porous medical device comprising:
(a) a sheet-like structural layer; and
(b) a multi-layered water-activatable adhesive component comprising a fibrinogen proximal to the structural layer, carboxymethyl cellulose (CMC) and a thrombin layer distal to the structural layer,
wherein the multi-layered, water-activatable adhesive component is attached to only one side of the sheet-like structural layer, and wherein a linker covalently connects the fibrinogen to the structural layer.

Importantly, the structural layer of (a) above is a particularly flexible and burst-resistant layer. Specifically, the sheet-like structural layer is a flexible plasma-based or a flexible cryoprecipitate-based film.

As such, the biodegradable, non-porous medical device of the first aspect combines several selected features, which provide for a unique combination of device characteristics, rendering the device particularly advantageous for the treatment of mild to severe and even arterial bleeds. Specifically, the distal thrombin layer - once in contact with blood (or any aqueous solution or exudate) - allows for the device to become an activator of clot formation via the patient's own coagulation system on the one hand and to be selectively affixed at the site of injury, trauma or surgery through the fibrinogen covalently connected to the sheet-like structural layer on the other. The fibrinogen covalently connected to the sheet-like structural layer itself, namely to proteins thereof (structural layer proteins; SLPs), participates in clot formation and thereby anchors the device - even against the withstand the streaming effect of a severe bleed. Given that the device is non-porous yet completely biodegradable, the anchored device is sufficiently stable, flexible and burst pressure resistant, such that it is capable not only of stemming the respective blood flow, but preferably, also to seal the site of injury, trauma or surgery.

Furthermore, it was found that the biodegradable, non-porous medical device according to the first aspect can advantageously be prepared without significant wastage of materials when following a particular sequence of production steps

Accordingly, in a second aspect, the present invention relates to a process for the production of a biodegradable, non-porous medical device according to the first aspect, comprising the steps of:
(a) providing a sheet-like structural layer;
(b) attaching a multi-layered water-activatable adhesive component to only on one side of the sheet-like structural layer, the attaching comprising
   (i) covalently connecting fibrinogen to only one side of the sheet-like structural layer such that a fibrinogen layer is formed; and
   (ii) depositing thrombin onto the fibrinogen layer under water-free conditions; and, optionally,
(c) drying the biodegradable, non-porous medical device.

In light of the above, the structural layer to be provided in step (a) of the process of the second aspect is:
- a plasma-based film comprising between 0.5 and 10 International Units (IU) of thrombin per ml of plasma, having a thickness ranging from 0.005 to 0.1 mm, and being characterized by a burst pressure of 50 to 1000 mm Hg; or
- a cryoprecipitate-based film comprising between 0.5 and 10 International Units (IU) of thrombin per ml of cryoprecipitate solution, has a thickness ranging from 0.005 to 0.1 mm, and is characterized by a burst pressure of 50 to 1000 mm Hg.

Typically, the residual moisture content of the structural layer is below 10%.

Usually, step (b)(i) comprises covalently connecting the fibrinogen to the one side of the structural layer using a suitable cross-linking agent. Regularly, the cross-linking agent is a multi-reactive NHS-ester such as an NHS-ester comprising several reactive groups capable of chemically reacting with amine groups. For example, the cross-linking agent is bis-N-succinimidyl diglycolic acid (DIG(NHS)₂; CAS-No 373614-12-1).

In particular embodiments of the second aspect step (b)(i) comprises:
- applying a layer of a multi-reactive cross-linking agent, which is capable of chemically reacting with amine groups, to only one side of the sheet-like structural layer under water-free conditions, preferably by applying glycerol and DIG(NHS)₂; and
- applying a fibrinogen layer to the one side of the sheet-like structural layer in the presence of water, preferably by applying an aqueous solution comprising carboxymethyl cellulose (CMC), fibrinogen and glycerol, wherein the presence of water allows for the cross-linking agent to react with amine groups present at the surface of the one side of the sheet-like structural layer and with amine groups of the fibrinogen such as to covalently connect the fibrinogen to the sheet-like structural layer;
- removing any remaining water; and

- step (b)(ii) comprises:
   - depositing thrombin in a solution of 0.1% to 2% polyvinylpyrrolidone in dry ethanol onto the fibrinogen layer.

In a third aspect, the present invention relates to a biodegradable, non-porous medical device when prepared by the process according to the second aspect.

In a fourth aspect, the present invention relates to the biodegradable, non-porous medical device according to the first or third aspects for use as a hemostat, preferably as a hemostat to stop a mild to severe bleeding. Accordingly, in embodiments of the fourth aspect, the invention also relates to a method of stopping a mild to severe bleeding, comprising applying the biodegradable, non-porous medical device according to the first or third aspects as a hemostat.

In a fifth aspect, the present invention relates to the biodegradable, non-porous medical device according to the first or third aspects for use as a hemostat to stop an arterial bleeding. Accordingly, in embodiments of the fifth aspect, the invention also relates to a method of stopping an arterial bleeding, comprising applying the biodegradable, non-porous medical device according to the third or fourth aspect as a hemostat.

### FIGURES

Embodiments of the invention will now be described, by way of example only, with reference to the accompanying drawings in which:
- Fig. 1: schematically shows a biodegradable, non-porous medical device (1) comprising a structural layer (2) and a multi-layered water-activatable adhesive component (3) according to an embodiment of the present invention; relative thickness of structural layer (2) and adhesive component (3) is not to scale.
- Fig. 2: schematically illustrates the layering of the biodegradable, non-porous medical device (1) like the one shown in Fig. 1, relative thickness of layers is not to scale.
- Fig. 3: shows (a) the reaction scheme for the reaction between an NHS-ester and the primary amine of a protein, (b) the structural formula of bis-N-succinimidyl diglycolic acid (DIG(NHS)₂; CAS-No 373614-12-1) as well as (c) the DIG(NHS)₂-derived, non-reactive linker (7) covalently connecting fibrinogen (6) to proteins of the structural layer (SLP)(2) of the device (1) of the invention.
- Fig. 4: is a tabular and graphical representation of a temperature and humidity profile as applied during the drying and thermal treatment of Part 1 of Example 1.

### DETAILED DESCRIPTION OF THE INVENTION

In order to provide a clear and consistent understanding of the specification and items, and of the scope to be given such terms, the following definitions are provided.

### Definitions

In the context of the present disclosure, the term ***"non-porous",*** in general refers to structures or devices that have such a low degree of porosity such that they do not allow for transmission of liquids such as water or blood through the structure or device itself. Therefore, such structures or devices can act at sealants to stem the immediate flow of such liquids. While even non-porous structures or devices of the present disclosure may absorb liquids over time, in contrast to other hemostatic patches or sponges the non-porous structures or devices of the present disclosure are not configured to be absorbents in order to quench flowing liquids and are not prone to significant swelling.

In the context of the present disclosure, the term ***"sheet-like",*** in general means something that is of a thin, substantially planar form, such as in the sense of a *"sheet of paper".*

Similarly, in the context of the present disclosure, the term ***"film",*** in general means, thin, substantially planar, membranous structures, which can also be described as sheets. Notwithstanding, the term film in the context of the present disclosure expressly encompasses single-layered films or sheets as well as composite multi-layered films or sheets. Further, a film in the context of the present disclosure may comprise pharmaceutically-active agents either topically applied to the film, homogeneously distributed throughout the film or encapsulated between the film layers of a multi-layered film. The term "film" also encompasses structures that allow for the release of substances such as pharmaceutically-active agents from the film. As such, the term "film" in the context of the present disclosure also encompasses a thin, substantially planar, membranous scaffold upon which, or within which, pharmaceutically-active agents may be contained. Accordingly, the term film when used to describe embodiments of the present invention also encompasses plasma-based vessels or delivery devices for pharmaceutically-active agents comprised on or within the film.

In the context of the present disclosure, the term ***"water-activatable adhesive component"*** means a multilayered component of the device of the present invention that mediates affixation of the medical device of the present invention to the site of injury, trauma or surgery, where the device is to be used as a hemostat. The layer most distant to the structural layer of the device is a thrombin layer. It is the thrombin layer that mediates the adhesive properties of the adhesive component when rehydrated by an aqueous solution such as blood because in such an aqueous environment, the thrombin immediately acts upon both the fibrinogen contained in the blood itself but also in adhesive component proximal to the structural layer such as to initiate immediate fibrin clot formation, which affixes the medical device to the site of injury, trauma or surgery through the polymerization of fibrin molecules, which are - at least partially - covalently connected to the structural layer of the device.

In the context of the present disclosure, and in contrast to the conditions present when activating fibrin clot formation described directly above, the term ***"water-free conditions"*** means conditions without any substantial amount of water being present. Thereby, under such water-fee conditions chemical reactions reliant on the presence of water cannot occur. Water-free conditions for example exist in non-aqueous organic solvents. Examples of such organic solvents are alcohols such as dry ethanol or glycerol, dry acetone, dry dichloromethane, etc. Accordingly, reactive agents that typically become activated in the presence of water can be provided in a non-aqueous solvent, i.e. under water-free condition, such as, for example thrombin in a solution of 0.1% to 2% polyvinylpyrrolidone in dry ethanol.

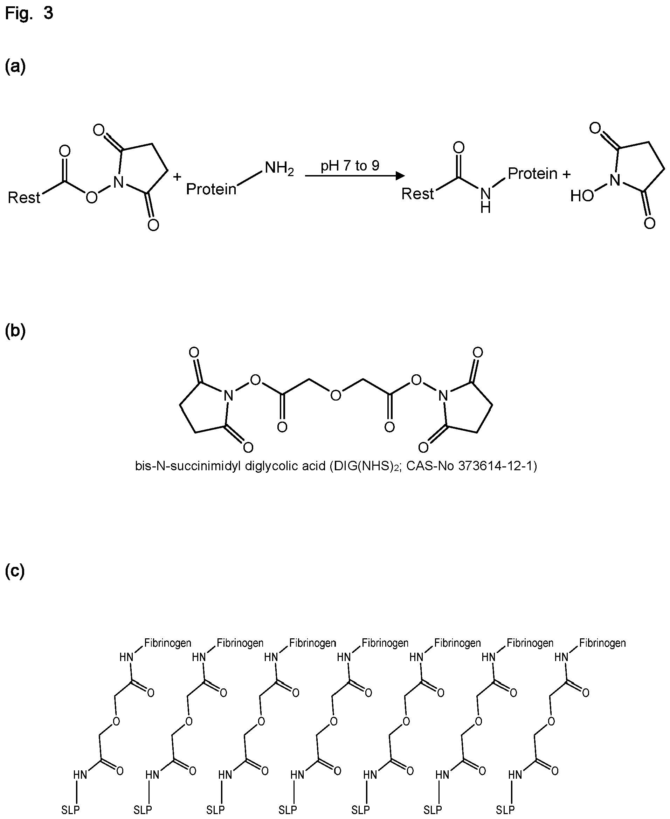

In the context of the present disclosure, the term ***"multi-reactive NHS-ester"*** means an ester of *N*-Hydroxysuccinimide (NHS), which comprises several, i.e. two or more, reactive groups capable of chemically reacting with amine groups. Typically, the several reactive groups are several NHS-ester groups interspaced on a backbone or linker structure. When the NHS-ester groups react with primary amine groups of peptides or proteins under physiologic pH conditions, a covalent connection between the backbone or linker and the primary amine of the respective peptide or protein is formed, while NHS itself is released (see Reaction Scheme of Figure 3). By selecting the appropriate reaction conditions, a covalent connection between the backbone of a multi-reactive NHS-ester with a first protein as well as with a second protein can be achieved such that the first and the second proteins are covalently connected to the same backbone or linker of the original multi-reactive NHS-ester and, as a result, are covalently connected to each other via this linker.

When all reactive ester groups have been exhausted, the first and second protein are then covalently connected to each other by the remaining ***"NHS-ester-derived, non-reactive linker".*** In this context, ***"non-reactive"*** refers to the linker's inability to react with amine groups, in particular with the primary amine groups, of peptides or proteins anymore.

In the context of the present disclosure, the term ***"flexible"*** means that a structure such as a plasma-based or cryoprecipitate-based film or structural layer is capable of bending, even in tight turns, without breaking. In particular, a flexible film or structural layer in the context of the present disclosure is capable of folding without breaking and, as such, the degree of flexibility can be expressed by way of fold numbers or fold endurance as defined below. Further, a structural layer's degree of flexibility can be expressed by the structural layer's burst pressure also as defined below.

In the context of the present disclosure, the term ***"plasma-based"**,* in general, means structures and materials such as the structural layer of the devices according to the present invention, which are produced from blood plasma as the source material. In particular, if the majority of the components of such structures or materials is plasma, they are understood to be plasma-based structures or materials.

In the context of the present disclosure, the term ***"cryoprecipitate-based"**,* in general, means structures and materials such as the structural layer of the devices according to the present invention, which are produced from cryoprecipitate as the source material. In particular, if the majority of the components of such structures or materials is cryoprecipitate, they are understood to be cryoprecipitate-based structures or materials. The cryoprecipitate itself is also obtained from plasma by methods well known in the field.

In the context of the present disclosure, the term ***"burst pressure"*** means the maximum pressure a structure can withstand before breaking or disrupting. In the context of the present disclosure, the term burst pressure particularly means the maximum pressure the medical device of the invention can withstand before breaking or disrupting. Typically, this characteristic of the device is provided through the particular structural layer, which is highly flexible, fully biodegradable and stable despite its minimal thickness.

In the context of the present disclosure the term ***"adhesion"*** refers to post-trauma, post-surgical or so-called surgery-induced tissue adhesions, which result from the formation of scar tissue. While adhesion is part of the normal healing response to tissue trauma, it can lead to severe post-surgical complications when a stable cross-connection between otherwise unconnected tissues arises as a result of adhesions.

In fact, more than 90% of patients develop peritoneal adhesions following abdominal or pelvic surgery. In most patients, the adhesion tissue is subsequently remodeled and ultimately dissolved. However, in millions of patients adhesions form between organs and/or tissues that are normally separate, such as the intestines, bowels, uterus, ovaries and other organs, causing dysfunction and other possibly severe complications. For example, within the abdominal cavity adhesions can lead to small bowel obstruction, female infertility and pain. In addition, adhesions often make subsequent surgery more difficult and limit subsequent treatment options. For example, adhesions often impede subsequent minimally invasive surgery.

Aside from their primary hemostat function, the medical devices of the present invention despite having an adhesive component on one side are suitable to act also as an ***"adhesion barrier"*** as they keep damaged tissue separate from the surrounding tissue, thereby preventing undesirable adhesion through the beneficial properties of the opposing side of the particular structural layer they comprise.

In the context of the present disclosure, the term ***"activator of the coagulation system"*** means substances, particles or agents that initiate coagulation/clotting. Such activators may initiate coagulation at any stage of the coagulation cascade and, as such, include agents acting on the intrinsic, extrinsic and common pathways of coagulation.

In the context of the present disclosure, the term ***"hemostat"*** means devices suitable to reduce and ultimately stop bleeding. In particular, the term encompasses plasma- and cryoprecipitate-based devices and films suitable to close blood vessels. As such, hemostats in the context of the present disclosure include plasma- and cryoprecipitate-based devices that can close blood vessels as a physical barrier as well as plasma- and cryoprecipitate-based devices that can deliver hemostatic agents to the site of bleeding to close the blood vessel as well as combinations thereof.

In addition to the above definitions, and unless the context clearly requires otherwise, throughout the description and the items, the words ***"comprise", "comprising",*** and the like are to be construed in an inclusive sense as opposed to an exclusive or exhaustive sense; that is to say, in the sense of "including, but not limited to".

Further, reference throughout this specification to ***"one embodiment", "some embodiments"*** or ***"an embodiment"*** means that a particular feature, structure or characteristic described in connection with the embodiment is included in at least one embodiment of the present invention. Thus, appearances of the phrases "in one embodiment", "in some embodiments" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

As used herein, unless otherwise specified the use of the ordinal adjectives **"*first"*, *"second", "third",*** etc., to describe a common object, merely indicate that different instances of like objects are being referred to, and are not intended to imply that the objects so described must be in a given sequence, either temporally, spatially, in ranking, or in any other manner.

As used herein, the term ***"exemplary"*** is used in the sense of providing examples, as opposed to indicating quality. That is, an ***"exemplary embodiment"*** is an embodiment provided as an example, as opposed to necessarily being an embodiment of exemplary quality.

### Biodegradable, non-porous medical device and methods for making and using the same

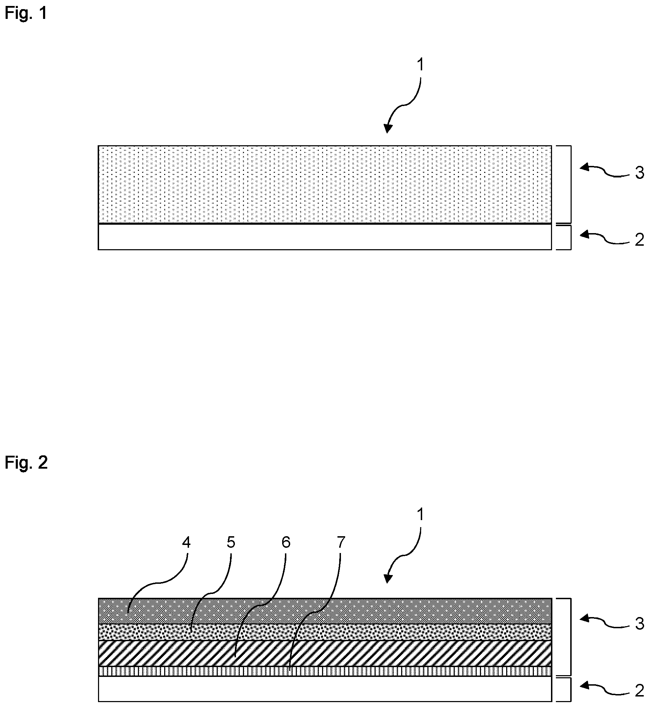

As described above, both the flexibility, elasticity and/or mechanical strength of the structural layer as well as the immediate fixation at the site of injury, trauma or surgery mediated by the specifically-developed adhesive component of the biodegradable, non-porous medical device of the present invention are important for such devices to be efficiently and successfully used as hemostats for stopping mild to severe bleedings and even for stopping arterial bleedings. Importantly, the non-porous medical device (1) of the present invention illustrated in Figure 1 provides the required flexibility, elasticity, mechanical strength as well as fixation properties required for the device to seal such bleeds, i.e. the flexibility necessary for the device to conform to the physiological topology of the site of the bleed and the fixation properties to withstand even arterial blood pressure and the streaming effects at the site of a bleed.

In addition, the devices of the present invention can seal surgical wounds not just to stop immediate bleeds but also to prevent postoperative leakage - not only of blood but also of fluids produced by the organs upon which the surgeon operated. For example, following liver surgery, postoperative biliary leakage is considered highly problematic as it may result in abdominal sepsis and even postoperative mortality. Similarly, pancreatic leakage following surgery can lead to severe complications. Therefore, it is of great importance that a medical device used as hemostat during such surgeries is configured to stay in place and seal the wound - at least for the period required for the respective tissue to heal sufficiently to prevent leakage.

To this end, the biodegradable, non-porous medical device of the present invention comprises:
(a) a sheet-like structural layer; and
(b) a multi-layered water-activatable adhesive component comprising fibrinogen proximal to the structural layer, carboxymethyl cellulose (CMC) and a thrombin layer distal to the structural layer,
wherein the multi-layered, water-activatable adhesive component is attached to only one side of the sheet-like structural layer, and wherein a linker covalently connects the fibrinogen to the structural layer.

Through their composition from substantially only endogenous components, the biodegradable, non-porous medical devices of the present invention are typically non-toxic, non-interfering with normal healing, non-interfering with immunological functions, applicable in various settings such as bleeding and anastomotic surgery and are, due to their flexibility, elasticity and high mechanical strength easy to handle and easy to apply.

As already indicated, these mechanical properties of the device of the present invention are largely provided by the structural layer. In instances where the structural layer is a flexible plasma-based or a flexible cryoprecipitate-based film, the film comprises between 0.5 and 10 International Units (IU) of thrombin per ml of plasma, has a thickness ranging from 0.005 to 0.1 mm, and is characterized by a burst pressure of 50 to 1000 mm Hg.

Typically, the residual moisture content of the structural layer is below 10%, in particular the residual moisture content is between 2 and 6%, such as between 3 and 5% or is 2%, 3%, 4%, 5%, 6%, 7%, 8% or 9%.

In this regard, it is important to note that the flexible, plasma-based or cryoprecipitate-based films when used as the structural layer in the devices of the present invention can have a thickness ranging from about 0.005 to 0.1 mm, or from about 0.005 to 0.09 mm, or from about 0.0075 to 0.08 mm, or from about 0.01 to 0.08 mm, or from about 0.01 to 0.07 mm, or from about 0.015 to 0.065 mm, or from about 0.02 to 0.06 mm, or from about 0.02 to 0.055 mm, or from about 0.02 to 0.05 mm, or from about 0.02 to 0.04 mm, or from about 0.02 to 0.03 mm, or about 0.01 mm, or about 0.02 mm, or about 0.03 mm, or about 0.04 mm, or about 0.05 mm, or about 0.06 mm. Notwithstanding, films with other thicknesses are also derivable by simply up- or down-scaling the volumes of the components used in the methods of the first and second aspects. Using such thin structural layers, allows for the devices of the present invention to be used to stop bleeds even where very little space is available and where using thicker material may lead to negative compression effects onto the adjacent tissues.

With respect to the beneficial mechanical strength, the flexible, plasma-based or cryoprecipitate-based films when used as the structural layer in the devices of the present invention are typically characterized by a burst pressure of about 50 to 1000 mm Hg. Burst pressure of such a film may be determined according to the Standard Test Method for Burst Strength of Surgical Sealants (ASTM-F 2392-04). The film is fastened in a fixture according to ASTM-F 2392-04 and the fixture is connected to a pump with a pressure transducer being attached inline between the pump and the burst pressure fixture. Pumping a fluid into the system increases the pressure until the film bursts. The pressure at film burst is corrected by atmospheric pressure and the so obtained burst pressure is indicated as [mm Hg]. Plasma-based or cryoprecipitate-based films when used as the structural layer in the devices of the present invention are typically characterized by a burst pressure of about 50 to 1000 mm Hg, or of about 100 to 1000 mm Hg, or of about 100 to 800 mm Hg, or of about 100 to 600 mm Hg, or of about 100 to 500 mm Hg, or of about 100 to 450 mm Hg, or of about 140 mm Hg, or of about 150 mm Hg, or of about 175 mm Hg, or of about 200 mm Hg, or of about 225 mm Hg, or of about 250 mm Hg, or of about 275 mm Hg, or of about 300 mm Hg, or of about 325 mm Hg, or of about 350 mm Hg, or of about 375 mm Hg, or of about 400 mm Hg. This strength is advantageous when the devices of the present invention are deployed in situations where high pressures act upon the device. Such pressures may be exerted by, e.g. the blood pressure building up when stemming an arterial bleed but also in situations where the device is subject to stretch, e.g. when applied to stem bleeds in/on contracting muscles, etc.

In the devices of the present invention, the thrombin layer comprises between about 15 and about 30 International Units (IU) of thrombin per cm², preferably between about 15 and 25 IU of thrombin per cm² such as about 15, 16, 17, 18, 19, 20, 21, 22, 23, 24, or 25 IU of thrombin per cm², of said sheet-like, structural layer. As already indicated above, the thrombin layer provides the adhesive component of the device with the ability to utilise both the endogenous fibrinogen present in the blood of the patient as well as the fibrinogen covalently connected to the structural layer, namely to a structural layer protein (SLP) to initiate fibrin clot formation and to affix the device at the site of injury, trauma or surgery upon contact with the blood.

Depending on the strength of adhesion required for the device, the amount of fibrinogen provided as part of the adhesive component of the biodegradable, non-porous medical device can be varied. In particular, the adhesive component may comprise between 5 and 20 mg of fibrinogen per cm², of said sheet-like, structural layer, preferably between 18 and 20 mg of fibrinogen per cm² or between 18 and 20 mg of fibrinogen per cm² or between 16 and 20 mg of fibrinogen per cm² or between 14 and 20 mg of fibrinogen per cm² or between 12 and 20 mg of fibrinogen per cm² or between 10 and 20 mg of fibrinogen per cm² or between 8 and 20 mg of fibrinogen per cm² or between 6 and 20 mg of fibrinogen per cm² or between 5 and 18 mg of fibrinogen per cm² or between 5 and 16 mg of fibrinogen per cm² or between 5 and 14 mg of fibrinogen per cm² or between 5 and 12 mg of fibrinogen per cm² or between 5 and 10 mg of fibrinogen per cm² or between 5 and 8 mg of fibrinogen per cm² or between 6 and 18 mg of fibrinogen per cm² or between 6 and 16 mg of fibrinogen per cm² or between 8 and 16 mg of fibrinogen per cm² or between 8 and 14 mg of fibrinogen per cm² or between 8 and 12 mg of fibrinogen per cm² or 10 mg of fibrinogen per cm².

The amounts of thrombin and fibrinogen may be varied to achieve various adhesive strengths and reactivities such the time until stable fibrin clot formation commences can be varied. Typically, the time period until a fibrin clot forms that is stable enough to hold the device in place at the site of injury, trauma or surgery is shorter than 5 minutes such as between 30 and 300 seconds or between 45 and 240 seconds or between 45 and 210 seconds or between 45 and 180 seconds or between 45 and 150 seconds or between 45 and 135 seconds or between 60 and 150 seconds or between 75 and 150 seconds or between 90 and 150 seconds or between 105 and 150 seconds or between 105 and 135 seconds or is about 120 seconds.

The amount of carboxymethyl cellulose (CMC) provided as part of the adhesive component of the biodegradable, non-porous medical device can also be adjusted to be between 5 and 20 mg of CMC per cm², of said sheet-like, structural layer, preferably between 18 and 20 mg of CMC per cm² or between 18 and 20 mg of CMC per cm² or between 16 and 20 mg of CMC per cm² or between 14 and 20 mg of CMC per cm² or between 12 and 20 mg of CMC per cm² or between 10 and 20 mg of CMC per cm² or between 8 and 20 mg of CMC per cm² or between 6 and 20 mg of CMC per cm² or between 5 and 18 mg of CMC per cm² or between 5 and 16 mg of CMC per cm² or between 5 and 14 mg of CMC per cm² or between 5 and 12 mg of CMC per cm² or between 5 and 10 mg of CMC per cm² or between 5 and 8 mg of CMC per cm² or between 6 and 18 mg of CMC per cm² or between 6 and 16 mg of CMC per cm² or between 8 and 16 mg of CMC per cm² or between 8 and 14 mg of CMC per cm² or between 8 and 12 mg of CMC per cm² or can be 10 mg of CMC per cm².

In the embodiments of the first aspect, CMC is advantageously located between the fibrinogen and thrombin molecules such as to prevent undesired commencement of clotting should the water content of the device inadvertently increase such as due to high humidity conditions during storage etc. Thereby stabilizing the adhesive component until application to a site of injury, trauma or surgery. Importantly, however, once the device is deployed *in situ,* the CMC layer provides another advantageous property due to its inherent stickiness when hydrated. Therefore, once hydrated through contact with blood (or any aqueous solution or exudate), the CMC provides the advantage that the device will immediately adhere to the site of injury, trauma or surgery to some degree when applied. This makes it easier for the surgeon to hold the device in place for the short time required for fibrin clot fixation to commence, which ultimately fixates the device at the site of injury, trauma or surgery. Notwithstanding, and contrary to the fixation of the device mediated through fibrin clot formation, the initial CMC-mediated adherence still allows the surgeon to adjust the location of the device *in situ.*

As such, and as already indicated above, it is the one side of the sheet-like structural layer, with which the adhesive component is associated, which mediates fixation of the device when placed at a site of application, preferably at a site of injury, trauma or surgery.

However, while the side of the device, which is configured to affix the device at the at a site of injury, trauma or surgery, the opposing side of the sheet-like structural layer is not only not adhesive but - through the beneficial properties of the specific structural layer, e.g. the beneficial properties of the plasma-based or cryoprecipitate-based films used as the structural layer - provides the device with an adhesion barrier function.

The composition and degree of cross-linking between the components of the plasma- or cryoprecipitate-based films when used as structural layer, predominantly the degree of cross-linking between the fibrin polymers, influences the degradation speed of the device as such. Specifically, a high degree of cross-linking correlates with slow degradation, and a low degree of cross-linking correlates with fast degradation. Accordingly, controlling the degree of cross-linking within the structural layer itself allows for the control of the rate or speed of degradation and therefore allows for the device to be further customized according to its application.

For example, if the device is to be used as a hemostat during liver surgery but is also required to prevent biliary leakage subsequent to the surgery, the device should only lose its structural integrity, i.e. degrade, within 14 to 42 days of implantation such as within 14 to 35 days of implantation or within 18 to 35 days of implantation or within 21 to 35 days of implantation within 21 to 32 days of implantation or within 21 to 30 days of implantation or within 22 to 30 days of implantation or within 24 to 30 days of implantation or within 26 to 30 days of implantation or within 27 to 29 days of implantation or within 28 days of implantation.

When a flexible plasma-based or cryoprecipitate-based film is used as the structural layer of the device of the present invention, it is typically prepared from liquid plasma and the cryoprecipitate of such plasma, respectively, where the plasma may be human or animal plasma. For example, the plasma is Fresh Frozen Plasma (FFP) or is pathogen-safe plasma. FFP can be obtained from a blood bank. However, plasmas obtained from a professional plasma manufacturer such as CLS Behring, Baxalta or Octapharma are typically preferred as they provide much higher pathogen safety due to pathogen removal and inactivation methods incorporated in their production processes. Plasmas from plasma manufacturers are usually pooled and pathogen-safe plasmas.

In this context, pathogen-safe plasma is understood to have been subjected to viral inactivation treatment, to have been pasteurized, to have been radiated, and/or to have been nano-filtered. Typically, the pathogen-safe plasma being used as source material for films used as the structural layer of the device of the present invention has been subjected to solvent/detergent treatment (S/D treatment) to inactivate any viral pathogens possibly contained therein.

Importantly, given the relatively small amounts of blood plasma or cryoprecipitate needed to prepare the structural layer of the device of the present invention, devices from a patient's own plasma or cryoprecipitate can be prepared in advance of a planned surgery, where a surgeon may anticipate that a device may become required to be used as a hemostat or an anti-adhesion barrier.

As indicated above, it was surprisingly found that the biodegradable, non-porous medical device of the present invention having the above-described characteristics and features can advantageously be prepared by the process of the second aspect of the present invention mentioned above, i.e. that the ability of the device to be affixed to the site of injury, trauma or surgery is a function of the particular process of production of the device, and even more particularly by the specific configuration of the adhesive component of the device.

Namely, in the second aspect, the present invention relates to a process for the production of a biodegradable, non-porous medical device according to the first aspect, comprising the steps of:
(a) providing a sheet-like structural layer;
(b) attaching a multi-layered water-activatable adhesive component to only on one side of the sheet-like structural layer, the attaching comprising
   (i) covalently connecting fibrinogen to the only one side of the sheet-like structural layer such that a fibrinogen layer is formed; and
   (ii) depositing thrombin onto the fibrinogen layer under water-free conditions; and, optionally,
(c) drying the biodegradable, non-porous medical device.

In light of the above, the structural layer to be provided in step (a) of the process of the second aspect is:
- a plasma-based film comprising between 0.5 and 10 International Units (IU) of thrombin per ml of plasma, having a thickness ranging from 0.005 to 0.1 mm, and being characterized by a burst pressure of 50 to 1000 mm Hg; or
- a cryoprecipitate-based film comprises between 0.5 and 10 International Units (IU) of thrombin per ml of cryoprecipitate solution, has a thickness ranging from 0.005 to 0.1 mm, and is characterized by a burst pressure of 50 to 1000 mm Hg.

In embodiments of the second aspect, in step (a), the structural layer may be provided on a supporting sheet. If the structural layer is provided on a supporting sheet, the structural layer rests on the supporting sheet with its other side, i.e. with the side opposite to the side to which the multi-layered water-activatable adhesive component is attached. As such, during step (b), care must be taken with respect to the orientation of the structural layer, i.e. care has to be taken that multi-layered water-activatable adhesive component is indeed attached to the structural layer and not accidently applied to the supporting sheet. To facilitate visual recognition of the correct orientation of the structural layer, the supporting sheet may contain indicia.

In instances where the structural layer is provided on the supporting sheet, the supporting sheet, however, is not required for the device's stability or integrity *per se.* Instead, the supporting sheet simply facilitates handling of the structural layer during the manufacturing process of the device, including the final stages of manufacture such as radiation sterilization, or cutting and packaging of the final device.

In particular, the supporting sheet can provide support during the cutting of the device such that each cut-out can be stored as an independent device (again, optionally with the supporting sheet) in a sealed bag with silica gel. As already indicated above, the supporting sheet may contain indicia, which not only facilitate visual recognition of the correct orientation of the device during its production process but also during its use. For example, the indicia may assist quick and easy identification of the adhesive side of the device when removing the device from its packaging in the operating theatre.

In particular embodiments of the process of the second aspect, step (b)(i) comprises covalently connecting the fibrinogen to the one side of the structural layer using a suitable cross-linking agent. Typically, the cross-linking agent is a multi-reactive NHS-ester such as an NHS-ester comprising several reactive groups capable of chemically reacting with amine groups. Generally, the several reactive groups are several NHS-ester groups interspaced on a backbone or linker structure. In a preferred embodiment the cross-linking agent is bis-N-succinimidyl diglycolic acid (DIG(NHS)₂; CAS-No 373614-12-1).

When the NHS-ester groups react with primary amine groups of peptides or proteins under physiologic pH conditions, a covalent connection between the backbone or linker and the primary amine of the respective peptide or protein is formed, while NHS itself is released (see Reaction Scheme of Figure 3(a)).

By selecting the appropriate reaction conditions, i.e. by only partially reacting the available reactive ester groups, a covalent connection between the backbone of a multi-reactive NHS-ester and a first protein can be achieved without completely exhausting all reactive ester groups. Subsequently, a second primary amine reaction can be performed to also covalently connect a second protein to the same backbone or linker of the original multi-reactive NHS-ester. Alternatively, the covalent connection between the respective first and second proteins can occur simultaneously, if both proteins are allowed to react with the NHS-ester groups simultaneously. The stoichiometry of the reagents, i.e. of first and second protein compared to the reactive groups of the NHS linker, will then be determinants of the reaction. In the context of the present invention, it is desirable that, when all reactive ester groups have been exhausted, the first and second proteins are covalently connected to each other by the remaining NHS-ester-derived, non-reactive linker.

As such, in step (b)(i) of the above process primary amines of fibrinogen and of the proteins of the structural layer proteins (SLPs) react with the reactive NHS-ester groups of the multi-reactive NHS-ester such that ultimately, fibrinogen is covalently connected, i.e. cross-linked, to proteins of the structural layer via the non-reactive linker remaining from the original NHS-ester used (see Figure 3(c)).

In view of the above, it will be appreciated that step (b)(i) may comprise:
- applying a layer of a multi-reactive cross-linking agent, which is capable of chemically reacting with amine groups, to only one side of the sheet-like structural layer under water-free conditions;
- applying a fibrinogen layer to the one side of the sheet-like structural layer in the presence of water, wherein the presence of water allows for the cross-linking agent to react with amine groups present at the surface of the one side of the sheet-like structural layer and with amine groups of the fibrinogen such as to covalently connect the fibrinogen to the sheet-like structural layer;
- removing any remaining water; and
in step (b)(ii) of the process described above, further:
- depositing thrombin in a solution of 0.1% to 2% polyvinylpyrrolidone in dry alcohol such as ethanol onto the fibrinogen layer.

Preferably, the multi-reactive cross-linking agent is DIG(NHS)₂, which is applied as DIG(NHS)₂ in glycerol and the fibrinogen layer is applied via an aqueous solution comprising carboxymethyl cellulose (CMC) and fibrinogen in glycerol.

In some embodiments of the process of the second aspect, the thrombin is deposited onto the fibrinogen layer in a solution of 0.1% to 2% polyvinylpyrrolidone in alcohol, preferably ethanol, or in a solution of 0.2% to 2% polyvinylpyrrolidone in alcohol, preferably ethanol, or in a solution of 0.4% to 2% polyvinylpyrrolidone in alcohol, preferably ethanol, or in a solution of 0.6% to 2% polyvinylpyrrolidone in alcohol, preferably ethanol, or in a solution of 0.8% to 2% polyvinylpyrrolidone in alcohol, preferably ethanol, or in a solution of 1.0% to 2% polyvinylpyrrolidone in alcohol, preferably ethanol, or in a solution of 1.2% to 2% polyvinylpyrrolidone in alcohol, preferably ethanol, or in a solution of 1.4% to 2% polyvinylpyrrolidone in alcohol, preferably ethanol, or in a solution of 1.6% to 2% polyvinylpyrrolidone in alcohol, preferably ethanol, or in a solution of 1.8% to 2% polyvinylpyrrolidone in alcohol, preferably ethanol, or in a solution of 0.1% to 0.3% polyvinylpyrrolidone in alcohol, preferably ethanol, or in a solution of 0.1% to 0.5% polyvinylpyrrolidone in alcohol, preferably ethanol, or in a solution of 0.1% to 0.7% polyvinylpyrrolidone in alcohol, preferably ethanol, or in a solution of 0.1% to 0.9% polyvinylpyrrolidone in alcohol, preferably ethanol, or in a solution of 0.1% to 1.1% polyvinylpyrrolidone in alcohol, preferably ethanol, or in a solution of 0.1% to 1.3% polyvinylpyrrolidone in alcohol, preferably ethanol, or in a solution of 0.1% to 1.5% polyvinylpyrrolidone in alcohol, preferably ethanol, or in a solution of 0.1% to 1.7% polyvinylpyrrolidone in alcohol, preferably ethanol, or in a solution of 0.1% to 1.9% polyvinylpyrrolidone in alcohol, preferably ethanol, or in a solution of 0.2% to 1.8% polyvinylpyrrolidone in alcohol, preferably ethanol, or in a solution of 0.4% to 1.6% polyvinylpyrrolidone in alcohol, preferably ethanol, or in a solution of 0.6% to 1.4% polyvinylpyrrolidone in alcohol, preferably ethanol, or in a solution of 0.8% to 1.2% polyvinylpyrrolidone in alcohol, preferably ethanol, or in a solution of 0.3% polyvinylpyrrolidone in alcohol, preferably ethanol,

### Examples

The invention is further described by the following non-limiting Examples.

### Example 1 - Producing biodegradable, non-porous medical device

### Part 1 - Preparation of a suitable sheet-like structural layer

### Prepare:

- 2M CaCl₂ in Water for Injection (WFI)
- 100 IU/mL Thrombin in WFI
- 10% Glycerol in WFI

Frozen plasma is thawed at 37°C in a water bath. Defrosting is finished when no more fibers and/or frozen pieces are visible in the container. In case there are some fibers, let the thawing process continue for some time, after reaching 37°C the fibers should be dissolved.

### Mixing and clotting:

For each sheet-like structural layer to be produced,
- An impermeable supporting sheet, preferably a plastic sheet, is placed inside an about 10 × 10 cm mold. The corners of the supporting sheet may have to be cut to size such as to ensure appropriate fit with the mold to be used, e.g. the corners may have to be rounded. The more precisely the supporting sheet fits into the mold, the less likely it is that the sheet will fully or partially separate from the structural layer during the clotting process applied during preparation of the structural layer.
- 100 g (± 1 g) of thawed plasma is transferred into a beaker.
- 1.0 mL of the prepared 2M CaCl₂ solution is added.
- Solution is gently mixed by swirling the beaker, to get a homogeneous solution.
- 1.0 mL of the freshly prepared or thawed 100 IU/mL thrombin solution is added.
- Solution is again gently mixed by swirling the beaker for 20 - 30 seconds. As soon as the solution clots, it must be transferred into the prepared mold and on top of the supporting sheet.

Upon transfer into the mold, the clotting process should last for at least 60 minutes.

### Pressing and Framing

- After 60 minutes clotting time, the mold is transferred upside-down, i.e. with the supporting sheet on top, into a press. The press actuates a punch, which has a punch area of about 10.2 cm² and applies downward pressure onto a pressing plate, which covers the entire area of the sheet-like structural layer within the mold. In the present example, considering that the area the pressing plate is slightly smaller than the total area of the 10×10 cm mold, the area upon which the pressure is applied is just under 100 cm², namely about 98 cm².
- A pressure sensor able to measure the pressure applied to the punch area is used to slowly, i.e. such as over a period of up to two minutes, increase pressure up to about 50 psi (about 345 kPa) is reached. Given the ratio between the punch area of about 10.2 cm² and the pressing plate, area of about 98 cm², the pressing plate ultimately exerts a pressure of about 5.2 psi (36 kPa) onto the sheet-like structural layer. This pressure is maintained until no more liquid is squeezed out.
- The punch pressure is then further increased to a pressure between 90 and 100 psi (between about 620 and 690 kPa; exerting a pressure of between about 9.4 psi (about 64.8 kPa) and 10.4 (about 69 kPa) onto the sheet-like structural layer ), which is maintained for 1 minute.
- Afterwards the pressure is slowly released.
- The supporting sheet may now be removed from or left on the so-formed structural layer, which is is fixed in a frame.
- The structural layer with the metal frame (and optionally with the supporting sheet) is then transferred into a 10% glycerine bath.

### Washing

The structural layer is washed three times for 20 minutes per wash in a washing solution of 10% glycerine. The washing solution must be renewed after each wash.

With each wash step, the solution should become less coloured. After the first step it is usually yellow, after the last one colourless.

### Drying and Thermal Treatment

- Transfer the structural layer (incl. metal frame) into a climate chamber, with preferably 37°C and 25%Rh (Relative humidity).
- Let the structural layer dry for about 24 hours.
- Set the temperature of the chamber to 100°C, with a temperature slope of 32°C/h, and lower the humidity to 10%Rh.
- After the temperature is reached hold for 120 minutes.
- Set the temperature of the chamber to 37°C and humidity to 25%Rh. Set the temperature slope to 32°C/h.

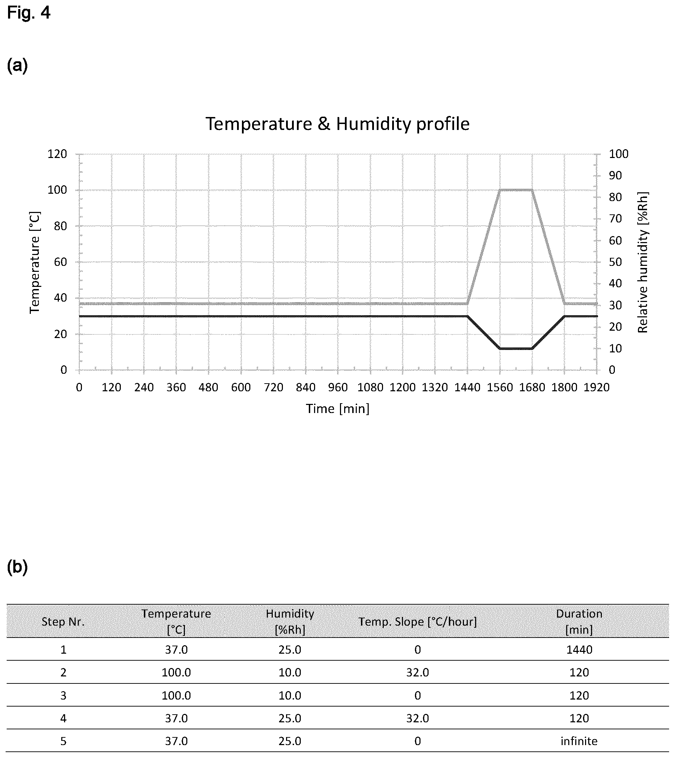

Figure 4 provides a tabular and graphical representation of the temperature and humidity profile. The residual moisture content of the structural layer is below 10%, in particular the residual moisture content is between 2 and 6%.

### Part 2 - Applying first ingredients of adhesive component

### Aqueous Fibrinogen/CMC solution

For a structural layer as that prepared in Part 1 of the present Example, prepare the following solution:
- 55.0 g 2% Fibrinogen in 20mM arginine/10 mM citric acid buffer, pH 6.8 to 7.2.
- 1.44 g Glycerol
- 1.10 g Carboxymethyl cellulose (CMC), high viscosity

Typically, and to achieve sufficient homogeneity, the solution is stirred overnight. As the solution is relatively viscous, it should not be stirred too vigorously, as otherwise too many air bubbles form, which may remain even after drying of the fibrinogen layer leading to undesirable appearance.

### NHS-ester layer

For each structural layer of about 100 cm² to be covered with 100 mg bis-N-succinimidyl diglycolic acid (DIG(NHS)₂; CAS-No 373614-12-1), 100 mg DIG(NHS)₂ are weighed directly onto the dried structural layer and then evenly distributed in a few drops of pure, water-free Glycerol. If the supporting sheet is still present care has to be taken with respect to the orientation of the structural layer to be coated, i.e. care has to be taken that the subsequent layers are applied to the structural layer and not the supporting sheet. To facilitate visual recognition of the correct orientation, the supporting sheet may contain indicia.

### Co valently connecting fibrinogen to structural layer

After the NHS-esterlayer has been applied, the fibrinogen is provided onto the NHS-ester layer in the aqueous fibrinogen/CMC solution - preferably without any delay.

To achieve distribution of about 10 mg of fibrinogen and 10 mg of CMC per cm² of structural layer, the 10x10 cm structural layer must be coated with 52.31 g of the homogeneous aqueous Fibrinogen/CMC solution.

Fibrinogen/CMC coated devices should be dried for at least 24 hours in a 37°C climate chamber, with 25%Rh.

Afterwards, they are again transferred to a desiccator, filled with silica gel, where they are further dried for at least 24 hours, ideally 48 hours.

While the covalent connection of the fibrinogen to the structural layer begins with the provision of the aqueous fibrinogen/CMC solution onto the structural layer, upon which the NHS-layer has been applied, this cross-linking reaction continues during the drying of the Fibrinogen/CMC coated devices.

### Part 3 - Completion of adhesive component

### Preparing the suspension

For each intermediate device to be covered with 2000 International Units (IU) of thrombin, i.e. with about 20 lUs per cm² of the device, thrombin powder is first pulverized with the help of a mortar and pestle to a fine powder and then suspended in a 0.3% polyvinylpyrrolidone in ethanol solution to a concentration of 1000 IU/mL.

### Coating

Before each transfer to the intermediate device, the suspension is freshly mixed using a vortex so that the particles are homogenous in suspension. 2 mL of the suspension are used for each intermediate device. The suspension is transferred to the device using a pipette and as slowly as possible so that the suspension can be distributed over the entire patch evenly.

### Drying

After an intermediate device is coated with the thrombin suspension, it is stored in the desiccator for about 1 to 2 days. Subsequent to this drying step, the device of the present invention is ready for use. In instances where the supporting sheet was maintained throughout the production process, the dry device still rests on the supporting sheet. While not required for the devices stability or integrity *per se,* the supporting sheet can still facilitate handling of the final devices.

That said, according to the present example, at this stage, each device prepared is still 10x10 cm in size. As such, it can now be cut into the desired size, such as 3x3 cm. The supporting sheet can provide support during the cutting of the device and each cut-out can be stored as an independent device (again, optionally with the supporting sheet) in a sealed bag with silica gel. As already indicated above, the supporting sheet may contain indicia, which not only facilitate visual recognition of the correct orientation of the device during its production process but also during its use.

### Example 2 - Burst pressure resistance

### Plasma-based structural layers

### Structural layer A

A highly flexible plasma-based structural layer was prepared by mixing 60 ml plasma with either 580-620 µl thrombin (300 IU/ml) or 580-620 µl calcium solution (2 M Ca²⁺) or 580-620 µl thrombin (300 IU/ml) together with about 580-620 µl calcium solution (2 M Ca²⁺), subsequently incubating the mixture at 25-37°C for about 15 minutes, in a 4"x4" (about 10x10 cm) mold and pressurizing the obtained plasma clot in the mold via a pressing plate such that a pressure of about 4.57 psi (31.5 kPa) is exerted onto the structural layer for about 60 seconds.

Finally, the obtained structural layer was dried in a laminar hood overnight. This structural layer is indicated in the below Table 1 as "Structural Layer A (dry)".

Another Structural Layer A (dry) prepared as described directly above, was subsequently soaked in 10 % glycerol for 10 minutes. This soaked Structural Laye A is indicated as "Structural Layer A (soaked)" in Table 1 below.

### Structural layer B

A highly flexible plasma-based structural layer was prepared by mixing 120 ml plasma with either 1180-1220 µl thrombin (300 IU/ml) or 1180-1220 µl calcium solution (2 M Ca²⁺) or 1180-1220 µl thrombin (300 IU/ml) together with about 1180-1220 µl calcium solution (2 M Ca²⁺), subsequently incubating the mixture at 25-37°C for about 15 minutes, in a 4"x4" (about 10x10 cm) mold and pressurizing the obtained plasma clot in the mold via a pressing plate such that a pressure of about 4.57 psi (31.5 kPa) is exerted onto the structural layer for about 60 seconds.

Finally, the obtained structural layer was dried in a laminar hood overnight. This structural layer is indicated in the below Table 1 as "Structural Layer B (dry)".

Another Structural Layer B (dry) prepared as described directly above, was subsequently soaked in 10 % glycerol for 10 minutes. This soaked Structural Layer B is indicated as "Structural Layer B (soaked)" in Table 1 below.

### Cryoprecipitate-based structural layers

### Structural layer C

A highly flexible plasma-based structural layer was prepared by mixing 100 ml cryoprecipitate with either 983.33-1016.67 µl thrombin (300 IU/ml) or 983.33-1016.67 µl calcium solution (2 M Ca²⁺) or 983.33-1016.67 µl thrombin (300 IU/ml) together with about 983.33-1016.67 µl calcium solution (2 M Ca²⁺), subsequently incubating the mixture at 25-37°C for about 15 minutes, in a 4"x4" (about 10x10 cm) mold and pressurizing the obtained plasma clot in the mold via a pressing plate such that a pressure of about 4.57 psi (31.5 kPa) is exerted onto the structural layer for about 60 seconds.

Finally, the obtained structural layer was dried in a laminar hood overnight. This structural layer is indicated in the below Table 1 as "Structural Layer C (dry)".

**Table 1 (Burst Pressure)**

| Structural Layer | Thickness [mm] | Burst Pressure [mmHg] | average Burst Pr. [mm Hg] | Burst Pr./mm Thickness |
|---|---|---|---|---|
| A (dry) | 0.03 | 186-218 | 198 | 6200-7267 |
| A (soaked) | | 149-162 | 156 | |
| B (dry) | 0.05-0.06 | 318-441 | 404 | 5300-8820 |
| B (soaked) | | 194-400 | 314 | |
| C (dry) | n.d. | 240-310 | n.d. | n.d. |

| | | | | |
|---|---|---|---|---|
| n.d. = not determined | | | | |

Many modifications and other embodiments of the invention set forth herein will come to mind to the one skilled in the art to which the invention pertains having the benefit of the teachings presented in the foregoing description and the associated drawings. Therefore, it is to be understood that the invention is not to be limited to the specific embodiments disclosed and that modifications and other embodiments are intended to be included within the scope of the appended items. Although specific terms are employed herein, they are used in a generic and descriptive sense only and not for purposes of limitation.

### List of reference signs

- 1: biodegradable, non-porous medical device
- 2: structural layer
- 3: multi-layered water-activatable adhesive component
- 4: thrombin
- 5: CMC
- 6: fibrinogen
- 7: NHS-ester-derived, non-reactive linker

The invention is further described by the following items:
1. A biodegradable, non-porous medical device (1) comprising:
   (a) a sheet-like structural layer (2); and
   (b) a multi-layered water-activatable adhesive component (3) comprising fibrinogen (6) proximal to said structural layer (2), carboxymethyl cellulose (CMC) (5) and a thrombin layer (4) distal to said structural layer,
   wherein said multi-layered, water-activatable adhesive component (3) is attached to only one side of said sheet-like structural layer (2), and wherein a linker (7) covalently connects said fibrinogen to said structural layer.
2. The biodegradable, non-porous medical device (1) of item 1, wherein said sheet-like structural layer (2) is a flexible plasma-based or a flexible cryoprecipitate-based film.
3. The biodegradable, non-porous medical device (1) of item 2, wherein said plasma-based film comprises between 0.5 and 10 International Units (IU) of thrombin per ml of plasma, has a thickness ranging from 0.005 to 0.1 mm, and is characterized by a burst pressure of 50 to 1000 mm Hg.
4. The biodegradable, non-porous medical device (1) of item 2, wherein said cryoprecipitate-based film comprises between 0.5 and 10 International Units (IU) of thrombin per ml of cryoprecipitate solution, has a thickness ranging from 0.005 to 0.1 mm, and is characterized by a burst pressure of 50 to 1000 mm Hg.
5. The biodegradable, non-porous medical device (1) of any one of items 1 to 4, wherein said linker (7) is an NHS-ester-derived, non-reactive linker.
6. The biodegradable, non-porous medical device (1) of item 5, wherein said NHS-ester-derived, non-reactive linker is a bis-N-succinimidyl diglycolic acid - derived ((DIG(NHS)₂-derived) linker.
7. The biodegradable, non-porous medical device (1) of any one of items 1 to 6, wherein said thrombin layer (4) comprises between about 15 and about 30 International Units (IU) of thrombin per cm², preferably between about 15 and 25 IU of thrombin per cm² or about 20 IU of thrombin per cm², of said sheet-like, structural layer (2).
8. The biodegradable, non-porous medical device (1) of any one of items 1 to 7, wherein said adhesive component (3) comprises:
   (a) between 5 and 20 mg of fibrinogen (6) per cm², of said sheet-like, structural layer (2), preferably between 8 and 12 mg of fibrinogen per cm² or 10 mg of fibrinogen per cm²; and
   (b) between 5 and 20 mg of CMC (5) per cm², of said sheet-like, structural layer (2), preferably between 8 and 12 mg of CMC per cm² or 10 mg of CMC per cm².
9. The biodegradable, non-porous medical device (1) of any one of items 1 to 8, wherein said one side of the sheet-like structural layer (2), with which the adhesive component (3) is associated, mediates fixation of the device (1) when placed at a site of application, preferably at a site of injury, trauma or surgery.
10. The biodegradable, non-porous medical device (1) of item 9, wherein the opposing side of said sheet-like structural layer (2) provides said device (1) with an adhesion barrier function.
11. A process for the production of a biodegradable, non-porous medical device (1) according to any one of the preceding items, comprising the steps of:
   (a) providing a sheet-like structural layer (2);
   (b) attaching a multi-layered water-activatable adhesive component (3) to only on one side of said sheet-like structural layer (2), said attaching comprising
      (i) covalently connecting fibrinogen (6) to said only one side of said sheet-like structural layer (2) such that a fibrinogen layer (6) is formed; and
      (ii) depositing thrombin (4) onto said fibrinogen layer under water-free conditions; and, optionally,
   (c) drying said biodegradable, non-porous medical device (1).
12. The process for the production of a biodegradable, non-porous medical device (1) according to item 11, wherein said structural layer (2) to be provided in step (a) is:
   - a plasma-based film comprising between 0.5 and 10 International Units (IU) of thrombin per ml of plasma, having a thickness ranging from 0.005 to 0.1 mm, and being characterized by a burst pressure of 50 to 1000 mm Hg; or
   - a cryoprecipitate-based film comprising between 0.5 and 10 International Units (IU) of thrombin per ml of cryoprecipitate solution, has a thickness ranging from 0.005 to 0.1 mm, and is characterized by a burst pressure of 50 to 1000 mm Hg.
13. The process for the production of a biodegradable, non-porous medical device (1) according to item 11 or item 12, wherein said step (b)(i) comprises covalently connecting said fibrinogen (6) to said one side of said structural layer (2) using a suitable cross-linking agent, preferably the cross-linking agent is a multi-reactive NHS-ester such as an NHS-ester comprising several reactive groups capable of chemically reacting with amine groups.
14. The process for the production of a biodegradable, non-porous medical device (1) according to item 13, wherein said cross-linking agent is bis-N-succinimidyl diglycolic acid (DIG(NHS)₂; CAS-No 373614-12-1).
15. The process for the production of a biodegradable, non-porous medical device (1) according to any one of items 11 to 14, wherein
   - said step (b)(i) comprises:
      - applying a layer of a multi-reactive cross-linking agent, which is capable of chemically reacting with amine groups, to only one side of said sheet-like structural layer (2) under water-free conditions, preferably by applying glycerol and DIG(NHS)₂; and
      - applying a fibrinogen layer (6) to said one side of said sheet-like structural layer (2) in the presence of water, preferably by applying an aqueous solution comprising carboxymethyl cellulose (CMC) (5), fibrinogen (6) and glycerol, wherein the presence of water allows for the cross-linking agent to react with amine groups present at the surface of said one side of the sheet-like structural layer (2) and with amine groups of said fibrinogen (6) such as to covalently connect said fibrinogen (6) to said sheet-like structural layer (2);
      - removing any remaining water; and
   - said step (b)(ii) comprises:
      - depositing thrombin (4) in a solution of 0.1% to 2% polyvinylpyrrolidone in dry ethanol onto said fibrinogen layer (6).

## Claims

1. A biodegradable, non-porous medical device (1) comprising:
(a) a sheet-like structural layer (2); and
(b) a multi-layered water-activatable adhesive component (3) comprising fibrinogen (6) proximal to said structural layer (2), carboxymethyl cellulose (CMC) (5) and a thrombin layer (4) distal to said structural layer,
wherein said multi-layered, water-activatable adhesive component (3) is attached to only one side of said sheet-like structural layer (2), and wherein a linker (7) covalently connects said fibrinogen to said structural layer.

2. The biodegradable, non-porous medical device (1) of claim 1, wherein said sheet-like structural layer (2) is a flexible plasma-based or a flexible cryoprecipitate-based film.

3. The biodegradable, non-porous medical device (1) of claim 2, wherein said plasma-based film comprises between 0.5 and 10 International Units (IU) of thrombin per ml of plasma, has a thickness ranging from 0.005 to 0.1 mm, and is **characterized by** a burst pressure of 50 to 1000 mm Hg.

4. The biodegradable, non-porous medical device (1) of claim 2, wherein said cryoprecipitate-based film comprises between 0.5 and 10 International Units (IU) of thrombin per ml of cryoprecipitate solution, has a thickness ranging from 0.005 to 0.1 mm, and is **characterized by** a burst pressure of 50 to 1000 mm Hg.

5. The biodegradable, non-porous medical device (1) of any one of claims 1 to 4, wherein said linker (7) is an NHS-ester-derived, non-reactive linker.

6. The biodegradable, non-porous medical device (1) of claim 5, wherein said NHS-ester-derived, non-reactive linker is a bis-N-succinimidyl diglycolic acid - derived ((DIG(NHS)₂-derived) linker.

7. The biodegradable, non-porous medical device (1) of any one of claims 1 to 6, wherein said thrombin layer (4) comprises between about 15 and about 30 International Units (IU) of thrombin per cm², preferably between about 15 and 25 IU of thrombin per cm² or about 20 IU of thrombin per cm^{2,} of said sheet-like, structural layer (2).

8. The biodegradable, non-porous medical device (1) of any one of claims 1 to 7, wherein said adhesive component (3) comprises:
(a) between 5 and 20 mg of fibrinogen (6) per cm^{2,} of said sheet-like, structural layer (2), preferably between 8 and 12 mg of fibrinogen per cm² or 10 mg of fibrinogen per cm²; and
(b) between 5 and 20 mg of CMC (5) per cm^{2,} of said sheet-like, structural layer (2), preferably between 8 and 12 mg of CMC per cm² or 10 mg of CMC per cm².

9. The biodegradable, non-porous medical device (1) of any one of claims 1 to 8, wherein said one side of the sheet-like structural layer (2), with which the adhesive component (3) is associated, mediates fixation of the device (1) when placed at a site of application, preferably at a site of injury, trauma or surgery.

10. The biodegradable, non-porous medical device (1) of claim 9, wherein the opposing side of said sheet-like structural layer (2) provides said device (1) with an adhesion barrier function.

11. A process for the production of a biodegradable, non-porous medical device (1) according to any one of the preceding claims, comprising the steps of:
(a) providing a sheet-like structural layer (2);
(b) attaching a multi-layered water-activatable adhesive component (3) to only on one side of said sheet-like structural layer (2), said attaching comprising
(i) covalently connecting fibrinogen (6) to said only one side of said sheet-like structural layer (2) such that a fibrinogen layer (6) is formed; and
(ii) depositing thrombin (4) onto said fibrinogen layer under water-free conditions; and, optionally,
(c) drying said biodegradable, non-porous medical device (1).

12. The process for the production of a biodegradable, non-porous medical device (1) according to claim 11, wherein said structural layer (2) to be provided in step (a) is:
- a plasma-based film comprising between 0.5 and 10 International Units (IU) of thrombin per ml of plasma, having a thickness ranging from 0.005 to 0.1 mm, and being **characterized by** a burst pressure of 50 to 1000 mm Hg; or
- a cryoprecipitate-based film comprising between 0.5 and 10 International Units (IU) of thrombin per ml of cryoprecipitate solution, has a thickness ranging from 0.005 to 0.1 mm, and is **characterized by** a burst pressure of 50 to 1000 mm Hg.

13. The process for the production of a biodegradable, non-porous medical device (1) according to claim 11 or claim 12, wherein said step (b)(i) comprises covalently connecting said fibrinogen (6) to said one side of said structural layer (2) using a suitable cross-linking agent, preferably the cross-linking agent is a multi-reactive NHS-ester such as an NHS-ester comprising several reactive groups capable of chemically reacting with amine groups.

14. The process for the production of a biodegradable, non-porous medical device (1) according to claim 13, wherein said cross-linking agent is bis-N-succinimidyl diglycolic acid (DIG(NHS)₂; CAS-No 373614-12-1).

15. The process for the production of a biodegradable, non-porous medical device (1) according to any one of claims 11 to 14, wherein
- said step (b)(i) comprises:
• applying a layer of a multi-reactive cross-linking agent, which is capable of chemically reacting with amine groups, to only one side of said sheet-like structural layer (2) under water-free conditions, preferably by applying glycerol and DIG(NHS)₂; and
• applying a fibrinogen layer (6) to said one side of said sheet-like structural layer (2) in the presence of water, preferably by applying an aqueous solution comprising carboxymethyl cellulose (CMC) (5), fibrinogen (6) and glycerol, wherein the presence of water allows for the cross-linking agent to react with amine groups present at the surface of said one side of the sheet-like structural layer (2) and with amine groups of said fibrinogen (6) such as to covalently connect said fibrinogen (6) to said sheet-like structural layer (2);
• removing any remaining water; and
- said step (b)(ii) comprises:
• depositing thrombin (4) in a solution of 0.1% to 2% polyvinylpyrrolidone in dry ethanol onto said fibrinogen layer (6).
